# EUROPEAN PATENT SPECIFICATION

(11) **EP 1 931 619 B1**
(45) Date of publication and mention of the grant of the patent: **24.06.2015**
(21) Application number: 06793833.2
(22) Date of filing: 26.09.2006
(51) Int. Cl.: C07C 229/36, C07C 227/18, C07C 67/10, C07C 69/00

(54) **METHANESULPHONIC ACID SALT OF DIHYDROPHENYLGLYCINE METHYL ESTER OR OF PHENYLGLYCINE METHYL ESTER**
METHANSULPHONSÄURESALZ VON DIHYDROPHENYLGLYCIN METHYL ESTER ODER PHENYLGLYCINE METHYL ESTER
SEL D'ACIDE MÉTHANESULPHONIQUE D'ESTER DE DIHYDROPHÉNYLGLYCINE MÉTHYLE OU D'ESTER DE PHÉNYLGLYCINE MÉTHYLE

(30) Priority: 29.09.2005 EP 05108997
(43) Date of publication of application: 18.06.2008
(73) Proprietor: DSM Sinochem Pharmaceuticals Netherlands B.V., 2613 AX Delft (NL)
(72) Inventor: BOESTEN, Wilhelmus Hubertus Joseph, NL-6132 BJ Sittard (NL); HEEMSKERK, Dennis, NL-6451 AD Schinveld (NL)
(74) Representative: De Vroom, Erik
(86) International application number: PCT/EP2006/066756
(87) International publication number: WO 2007/039522

(56) References cited:
- EP-A1- 1 541 546
- WO-A-2006/121648
- CH-A5- 621 770
- DE-A1- 4 311 424
- GB-A- 2 211 844
- US-A- 1 882 808
- US-A1- 2002 128 452
- US-A1- 2003 162 832
- US-A1- 2004 006 137
- US-A1- 2004 133 033
- L. DUHAMEL, J.-C. PLAQUEVENT: "Déracémisation par protonation énantiosélective. Application à un alpha-aminoacide, la phénylglycine" BULL SOC CHIM FR, vol. 2, no. 1-2, 1982, pages 75-83, XP009069103 cited in the application
- DATABASE BEILSTEIN Beilstein Institut zur Förderung der Chemischen Wissenschaften, Frankfurt am Main, DE; XP002389649 accession no. BRN: 3578671, 3579557 & AUST J CHEM, vol. 52, no. 5, 1999, pages 379-386,
- DATABASE BEILSTEIN Beilstein Institut zur Förderung der Chemischen Wissenschaften, Frankfurt am Main, DE; XP002389650 accession no. BRN 3791940 & PECK, FOLKERS: "Chem Penicillin" 1949, , PRINCETON
- DATABASE BEILSTEIN Beilstein Institut zur Förderung der Chemischen Wissenschaften, Frankfurt am Main, DE; XP002389651 accession no. BRN 3582436 & J MED CHEM, vol. 20, 1977, pages 510-515,
- DATABASE BEILSTEIN BEILSTEIN INSTITUTE FOR ORGANIC CHEMISTRY, FRANKFURT-MAIN, DE; XP002416724 retrieved from XFIRE Database accession no. brn 9022452 & J ORG CHEM, vol. 66, no. 23, 2001, pages 7925-7929,
- DATABASE BEILSTEIN BEILSTEIN INSTITUTE FOR ORGANIC CHEMISTRY, FRANKFURT-MAIN, DE; XP002416725 retrieved from XFIRE Database accession no. brn 4343308 & LIEBIGS ANN CHEM, vol. 2, 1991, pages 165-170,
- DATABASE BEILSTEIN BEILSTEIN INSTITUTE FOR ORGANIC CHEMISTRY, FRANKFURT-MAIN, DE; XP002416726 retrieved from XFIRE Database accession no. brn 4345924 & LIEBIGS ANN CHEM, vol. 2, 1991, pages 165-170,
- DATABASE BEILSTEIN BEILSTEIN INSTITUTE FOR ORGANIC CHEMISTRY, FRANKFURT-MAIN, DE; XP002416727 retrieved from XFIRE Database accession no. brn 4117753 & MEM FAC SCI KYUSHU UNIV SER C, vol. 5, 1963, pages 27-31,
- DATABASE BEILSTEIN BEILSTEIN INSTITUTE FOR ORGANIC CHEMISTRY, FRANKFURT-MAIN, DE; XP002416728 retrieved from XFIRE Database accession no. brn 4071823 & AUST J CHEM, vol. 20, 1967, page 2283,
- DATABASE BEILSTEIN BEILSTEIN INSTITUTE FOR ORGANIC CHEMISTRY, FRANKFURT-MAIN, DE; XP002416729 retrieved from XFIRE Database accession no. brn 3799847 & J AM CHEM SOC, vol. 76, 1954, page 6203,
- DATABASE BEILSTEIN BEILSTEIN INSTITUTE FOR ORGANIC CHEMISTRY, FRANKFURT-MAIN, DE; XP002416730 retrieved from XFIRE Database accession no. brn 3642668 & J PHARM SCI, vol. 79, no. 5, 1990, pages 447-452,
- DATABASE BEILSTEIN BEILSTEIN INSTITUTE FOR ORGANIC CHEMISTRY, FRANKFURT-MAIN, DE; XP002416731 retrieved from XFIRE Database accession no. brn 3579557 & AUST J CHEM, vol. 52, no. 5, 1999, pages 379-386,
- DATABASE BEILSTEIN BEILSTEIN INSTITUTE FOR ORGANIC CHEMISTRY, FRANKFURT-MAIN, DE; XP002416732 retrieved from XFIRE Database accession no. brn 6465033 & BULL CHEM SOC JPN, vol. 57, no. 11, 1984, pages 3203-3209,
- DATABASE BEILSTEIN BEILSTEIN INSTITUTE FOR ORGANIC CHEMISTRY, FRANKFURT-MAIN, DE; XP002416733 retrieved from XFIRE Database accession no. brn 5207306 & J CHEM SOC PERKIN TRANS 1, 1987, pages 2285-2296,
- A. COSTANZO ET AL.: "Reactivity of 1-(2-Nitrophenyl)-5-Aminopyrazols under Basic Conditions and Synthesis of New 3-, 7-, and 8-Substituted Pyrazol[5,1-c][1,2,4]Benzotriazine 5-Oxides, as Benzodiazepine Receptor Ligands" J HETEROCYCL CHEM, vol. 31, 1994, pages 1369-1376, XP009077909
- DATABASE CA [Online] CHEMICAL ABSTRACTS SERVICE, COLUMBUS, OHIO, US 01 July 2005 BOAZ, NEIL W. ET AL: 'The Preparation of Single Enantiomer 2-Naphthylalanine Derivatives Using Rhodium-Methyl BoPhoz-catalyzed Asymmetric Hydrogenation' Retrieved from STN Database accession no. 143:44053
- DATABASE CA [Online] CHEMICAL ABSTRACTS SERVICE, COLUMBUS, OHIO, US 2004 MARUYAMA, NORIAKI ET AL: 'An Improved Process for the Large-Scale Preparation of Antirheumatic Agent MX-68' Retrieved from STN Database accession no. 141:412719
- DATABASE CA [Online] CHEMICAL ABSTRACTS SERVICE, COLUMBUS, OHIO, US HANESSIAN, STEPHEN ET AL: 'Asymmetric synthesis of L-azetidine-2-carboxylic acid and 3-substituted congeners - conformationally constrained analogs of phenylalanine, naphthylalanine, and leucine' Retrieved from STN Database accession no. 131:102512
- DATABASE CA [Online] CHEMICAL ABSTRACTS SERVICE, COLUMBUS, OHIO, US YAMASHITA, YUKIYOSHI ET AL: 'Method for preparing crystals of salts of amino compounds and acids' Retrieved from STN Database accession no. 139:69522
- DATABASE CA [Online] CHEMICAL ABSTRACTS SERVICE, COLUMBUS, OHIO, US SAWAI, YASUHIRO ET AL: 'Process for the preparation of optically active threo-.beta.-alkyltryptophan derivative and intermediate thereof' Retrieved from STN Database accession no. 142:297986

## Description

The present invention relates to a sulphonic acid salt of an amino acid alkyl ester, a process for the esterification of an organic acid with dialkylcarbonate, and the use of a sulphonic acid salt of an amino acid alkyl ester.

An alkanesulphonic acid salt of a ω-benzyl ester of amino dicarboxylic acid is known from US 04/0133033, in particular methanesulphonic acid salt of ω-benzyl ester of glutamic acid and aspartic acid. The alkanesulphonic acid salt of a ω-benzyl ester of amino dicarboxylic acid in US 04/0133033 was prepared by acid esterification by reacting the amino dicarboxylic acid with a benzyl alcohol in the presence of an alkane sulphonic acid.

A p-toluene sulphonic acid salt from phenylglycine ethyl ester and isopropyl ester is known from L. Duhamel & J.-C. Plaquevent, Bull. Soc. Chim, 1982, p. 75-83, wherein these compounds were prepared by acid esterification in the presence of benzene and ethanol or isopropanol, respectively.

It was found that the conversion of an amino acid into its corresponding ester in the esterification process as disclosed in US 04/0133033 and L. Duhamel & J.-C. Plaquevent, Bull. Soc. Chim, 1982, p. 75-83 was relatively low due to water formation in the esterification process.

The aim of the present invention is the provision of an alternative sulphonic acid salt of an amino acid alkyl ester, which can be obtained in a sufficiently high conversion. The aim is achieved with a methanesulphonic acid salt of dihydrophenylglycine or phenylglycine methyl ester (hereinafter also referred to as the amino acid alkyl ester), according to the present invention.

Surprisingly, the sulphonic acid salt of the amino acid alkyl ester was advantageously obtained in the process for the esterification according to the present invention in a high conversion and without the formation of water.

As used herein, the amino acid in the sulphonic acid salt of the amino acid alkyl ester according to the invention is an amino acid selected from the group consisting of dihydro-phenylglycine and phenylglycine.

It was found that when a salt, for instance a HCl salt, of dihydro-phenylglycine alkyl ester or of phenylglycine alkyl ester is used as activated side chain in the enzymatic synthesis of a ß-lactam antibiotic, for instance cephradine, or cephalexin,

cefaclor, and ampicillin respectively, the acylase used in the acylation reaction may be inhibited by side products present in the HCl salt of dihydro-phenylglycine alkyl ester or of phenylglycine alkyl ester.

Surprisingly, it was found that a sulphonic acid salt of dihydro-phenylglycine alkyl ester and of phenylglycine alkyl ester, according to the present invention does not comprise side products which inhibit the acylase used in the enzymatic acylation reaction in the synthesis of ß-lactam antibiotics.

The amino acid alkyl ester in the sulphonic acid salt according to the present invention may be present in any enantiomeric form, such as in the form of the pure *(D)-*enantiomer or the pure *(L)*-enantiomer or in the form of a racemic mixture. When the sulphonic acid salt of an amino acid alkyl ester, eg. phenylglycine alkyl ester or dihydro-phenylglycine alkyl ester, is to be used in the synthesis of a ß-lactam antibiotic, preferably the amino acid alkyl ester is present in the form of the *(D)*-enantiomer.

Alternatively, the amino acid alkyl ester may be present in the form of the *(L)-*enantiomer.

The amino acid alkyl ester may also be present in the form of a racemic mixture.

The alkyl in the sulphonic acid salt of an amino acid alkyl ester according to the invention is methyl.

The sulphonic acid in the sulphonic acid salt according to the invention is methane sulphonic acid.

The present invention also relates to a process for the esterification of an organic acid with dialkylcarbonate.

US 04/0133033 discloses a process for the preparation of ω-benzyl esters of amino dicarboxylic acid wherein an amino dicarboxylic acid is esterified with a benzyl alcohol in the presence of an alkanesulphonic acid. A disadvantage of the esterification process in US 04/0133033 is that one mole water is formed per mole of ω-benzyl alcohol, which suppresses the maximum conversion of amino dicarboxylic acid into the corresponding ester that can be achieved. Water will react with the formed ester bond, which shifts the equilibrium to the original reactants, i.e. the amino dicarboxylic acid and benzyl alcohol resulting in a decreased conversion.

The aim of the present invention is the provision of an alternative process for the esterification of an organic acid into the corresponding organic acid ester, wherein no water is formed, and which results in an increased conversion of the organic acid into its corresponding ester than disclosed in the prior art.

The aim is achieved according to the invention by a process for the esterification of dihydrophenylglycine or phenylglycine (hereinafter also referred to as the organic acid or as the amino acid) into the corresponding methyl ester (hereinafter also referred to as the organic acid alkyl ester or as the amino acid alkyl ester) comprising bringing the organic acid into contact with methanesulphonic acid (hereinafter also referred to as the strong acid) and a solution comprising dialkylcarbonate in a reaction mixture.

It is known that an amino acid can be esterified with a dialkylcarbonate under alkaline conditions. Under alkaline conditions however, the amino group of the amino acid is a strong nucleophile, which easily attacks the electrophile of dialkylcarbonate. This results in the formation of side-products such as carbamate, diketopiperazine (DKP), dipeptide, or polypeptide. In addition, under alkaline conditions racemisation of the amino acid and amino acid ester easily occurs.

It was surprisingly found that a high conversion of organic acid into its corresponding organic acid ester could be achieved and that no side products are formed in the esterification process according to the present invention.

Other advantages of the process according to the present invention are the following:
The initial water formed in the process for the esterification according to the present invention, immediately reacts with dialkylcarbonate to form carbondioxide and the corresponding alcohol. Therefore, the initial formed water cannot react with the ester bond in the amino acid alkyl ester.

In addition, no racemisation of the organic acid and the corresponding organic acid alkylester occurs.

Likewise, no reaction was found to take place between the amino group and dialkylcarbonate.

It was also found that esterification of the organic acid with a dialkylcarbonate under acid conditions according to the present invention resulted in an organic acid alkyl ester, which could be easily isolated from the reaction mixture.

The organic acid in the process according to the invention is an amino acid, *viz.* phenylglycine or dihydro-phenylglycine, for instance *(D)*-phenylglycine and *(D)*-dihydro-phenylglycine.

The organic acid and the corresponding organic acid ester in the process according to the present invention may be present in any suitable enantiomeric form, such as the *D*-enantiomer, the *L*-enantiomer or in the form of a racemic mixture.

In the process for the esterification according to the present invention, the organic acid, the strong acid and the dialkylcarbonate may be brought into contact with each other in any order and within any suitable period of time, depending on the stirrability of the reaction mixture, which is, amongst others, dependent on the concentration of the reactants, i.e. organic acid, dialkylcarbonate and strong acid in the reaction mixture. For instance, the organic acid may be brought into contact at once with the strong acid and the solution comprising dialkylcarbonate at the start of the reaction. The organic acid may also be brought into contact with the strong acid and the solution comprising dialkylcarbonate by dosing the strong acid during a period of time of between 1 to 120 min, preferably during a period of time of 5 to 90 min, preferably during a period of time of 10 to 60 min.

Methane sulphonic acid is used as strong acid in the process according to the present invention.

It is preferred that the strong acid in the reaction mixture is present in an amount equal to or larger than equimolar to the amino acid.

The solution comprising dialkylcarbonate in the esterification process according to the present invention may additionally comprise an alcohol. It was advantageously found that when an alcohol is present in the solution comprising dialkylcarbonate, a higher amount of amino acid salt was dissolved in the reaction mixture and the esterification reaction proceeded faster. The amino acid salt is formed when it is brought into contact with the strong acid in the reaction mixture prior to the esterification in the process according to the present invention.

When an alcohol is present in the solution comprising dialkylcarbonate, it is essential that the alcohol comprises an identical number of carbon atoms as the number of carbon atoms of the alkyl in dialkylcarbonate. For example, when alkyl in dialkylcarbonate is methyl or ethyl, the alcohol is methanol and ethanol, respectively.

The alkyl in dialkylcarbonate in the process according to the present invention may comprise any number of carbon atoms, for instance between 1 to 20 carbon atoms, preferably between 1 to 15, more preferably between 1 and 10 carbon atoms. Preferably, the alkyl in dialkylcarbonate comprises 1 to 6 carbon atoms. The alkyl may for example be methyl, ethyl, n-propyl, isopropyl, n-butyl, iso-butyl, pentyl, isopentyl, hexyl or isohexyl. Preferably, the alkyl is methyl or ethyl.

The esterification may be carried out at any suitable temperature in combination with suitable pressure and during any suitable period of time. In one embodiment, the esterification is carried out at atmospheric pressure and at a suitable temperature and during a suitable period of time. In another preferred embodiment, the esterification is carried out at a pressure above atmospheric, for instance at a pressure equal to or higher than 1 kg/m², more preferably at a pressure equal to or higher than 2 kg/m², even more preferably at a pressure equal to or higher than 3 kg/m². Since the reflux temperature of the reaction mixture is increased at a pressure above atmospheric, the reaction temperature of the esterification may be increased accordingly. The advantage of using an increased temperature at elevated pressure is that reaction time is decreased.

A pressure above atmospheric may be build up in a closed esterification reactor by the carbon dioxide (CO₂) which is produced as a result of the reaction of the water, liberated during the esterification reaction, with the dialkylcarbonate. Alternatively, the overpressure may be established in the closed esterification reactor by applying an overpressure using an external inert gas such as nitrogen (N₂), argon (Ar), etceteras. The esterification may for example comprise keeping the reaction mixture at a temperature below the reflux temperature of the dialkylcarbonate for at least 1 hour, preferably for at least 4 hours. Depending on the dialkylcarbonate, it was found that when the reaction mixture was kept at a temperature of below the reflux temperature of the dialkylcarbonate for a certain period of time at the start of the esterification, the reaction mixture remained stirrable. This was found to be in particularly advantageous when the concentration of organic acid in the reaction mixture is high in the case the organic acid is an amino acid. A high concentration of the amino acid may be between 10 and 50% w/w, for instance 20 to 40% or 25 to 35% w/w.

The reflux temperature is dependent on the dialkylcarbonate in the reaction mixture, and may suitably be below 90°C. It was found that by keeping the temperature of below about 90°C, racemization of an optically active organic acid, or organic acid alkylester did not occur.

As defined herein the reflux temperature is the boiling temperature of the dialkylcarbonate used in the esterification process.

In another preferred embodiment the esterification process comprises keeping the reaction mixture at a temperature of at least the reflux temperature of the dialkylcarbonate for at least 6 hours, preferably at least 24 hours, preferably between 48 hours and 7 days. It was found that when the temperature in the reaction mixture was kept at at least the reflux temperature of the dialkylcarbonate, the esterification reaction rate was increased.

During the esterification of the organic acid, an organic acid ester is formed. The organic acid ester may be present in dissolved form or may crystallise during esterification of the organic acid.

In a preferred embodiment of the present invention the organic acid ester is isolated from the reaction mixture.

Isolation of the organic acid ester from the reaction mixture may be performed in any suitable way. Preferably, isolation of the organic acid ester comprises distilling off part of the dialkylcarbonate or dialkylcarbonate and alcohol present in the reaction mixture. It was found that distilling off part of the dialkylcarbonate or dialkylcarbonate and alcohol present in the reaction mixture resulted in an improved yield of the organic acid ester.

Preferably, isolation of the organic acid ester also comprises adding fresh dialkylcarbonate to the reaction mixture, wherein the alkyl in the fresh dialkylcarbonate has the same number of carbon atoms as in the dialkylcarbonate used in the esterification process. Preferably, fresh dialkylcarbonate is added to the reaction mixture subsequent to distilling off part of the dialkylcarbonate or dialkylcarbonate and alcohol from the reaction mixture.

Isolation of the organic acid ester from the reaction mixture may be performed at any suitable temperature. The organic acid ester may for example be isolated at a temperature below the temperature at which the organic acid crystallises. Preferably, the organic acid ester is isolated at a temperature below 50°C, preferably below 40°C, more preferably below 35°C, more preferably below 30°C.

The isolation of the organic acid ester may also comprise adding a base to the reaction mixture, which neutralises the excess of strong acid present in the reaction mixture. Any organic or inorganic base may be used to neutralise the reaction mixture. Examples of suitable organic bases are triethylamine, diethylamine, diisopropylethylamine, and dicyclohexylamine. An inorganic base may for example be ammonia (NH₃) in gaseous form or in solution. Preferably, triethylamine is used to neutralise the reaction mixture.

The organic acid ester may be isolated from the reaction mixture in any suitable form, preferably in crystalline form. The organic acid ester is an amino acid ester and is preferably crystallised in the form of a salt. For instance, the amino acid ester may be crystallised in the form of a sulphonic acid salt in the esterfication process according to the present invention.

The organic acid ester in crystalline form may be further isolated from the reaction mixture by known methods in art, such as centrifugation and filtration.

In another preferred embodiment the organic acid ester in crystalline form may be dried at any suitable temperature and under any suitable pressure, for instance by drying under vacuum.

The present invention further relates to the use of a methanesulphonic acid salt of dihydrophenylglycine or phenylglycine methyl ester (hereinafter also referred to as the amino acid alkyl ester) in the synthesis of a ß-lactam antibiotic.

The sulphonic acid salt of an amino acid alkyl ester may be used as activated side chain in the enzymatic synthesis of a ß-lactam antibiotic, wherein the activated side chain may be enzymatically coupled to a suitable ß-lactam nucleus in the presence of an acylase, for instance as described in WO 2005/003367, WO00/00201, or EP 0771357.

A suitable ß-lactam nucleus is for instance 6-amino penicillanic acid (6-APA) 7-aminodesacetoxy cephalosporanic acid (7-ADCA), 7-amino cephalosporanic acid (7-ACA), 7-amino-3-[(Z)-1-propenyl]-3-(desacetoxymethyl)cephalosporanic acid, or 7-amino-3-chloro-cephalosporanic acid (7-ACCA).

In a preferred embodiment, the invention relates to the use of a sulphonic acid salt of *(D)*-phenylglycine alkyl ester in the synthesis of a ß-lactam antibiotic selected from the group consisting of cephalexin, cefaclor and ampicillin.

In another preferred embodiment, the invention relates to the use of a sulphonic acid salt of *(D)*-dihydro-phenylglycine alkyl ester in the synthesis of cephradine.

The alkyl in the use of a sulphonic acid salt of an amino acid alkyl ester in the synthesis of a ß-lactam antibiotic is methyl.

The sulphonic acid in the use of a sulphonic acid salt of an amino acid alkyl ester in the synthesis of a ω-lactam antibiotic is methane sulphonic acid.

The following examples are for illustrative purposes only and are not to be construed as being limited thereto.

### EXAMPLES

### Example 1

### Preparation of (D)-dihydro-phenylglycine methyl ester methanesulphonic acid salt (DHME.CH₃SO₃H)

0.5 mol (76.6 g) *(D)*-dihydro-phenylglycine (ee_{D} >99%) was suspended in a solution of 300 ml dimethylcarbonate and 10 ml methanol. Subsequently, 0.7 molmethanesulphonic acid was dosed to the suspension in 30 to 60 min at a temperature increasing from 30 to 75°C. After all the methane sulphonic acid was dosed to the reaction mixture, the temperature was kept at the reflux temperature of dimethylcarbonate, i.e. about 84 to 85°C. The temperature was kept for 5 hours at 84 to 85°C, after which the esterification reaction proceeded for 24 hours at a temperature of 82°C. The conversion of dihydro-phenylglycine into dihydro-phenylglycine methyl ester after 24 hours at 82°C was 99.5%.

Subsequently, 50 ml of methanol/dimethylcarbonate was distilled off from the reaction mixture at T= 82°C at atmospheric pressure. Thereafter, 150 ml of dimethylcarbonate was added to the reaction mixture and the reaction mixture was slowly cooled to a temperature of about 20°C. An amount of 0.17 moles of triethylamine was dosed to the reaction mixture in 30 min at 20°C. Subsequently, the reaction mixture was stirred at 20°C for 30 min. The reaction mixture was filtered by G3 filtration and the residue was washed 4 times with 50 ml dimethylcarbonate. The residue consisting of dihydro-phenylglycine methyl ester methanesulphonic acid salt (DHME.CH₃SO₃H) was dried under vacuum at 60°C under nitrogen, for 1 hour.

The yield of *(D)*-dihydro-phenylglycine methyl ester was 114.5 g (87%; ee_{D} ≥ 99%).

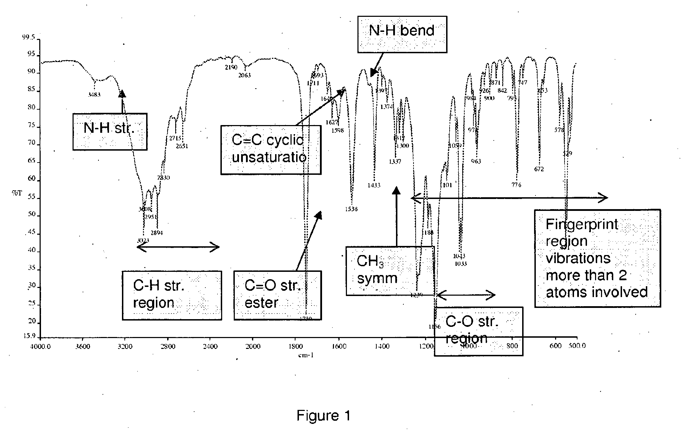

The melting point of DHME.CH₃SO₃H was 130°C as determined with a Buchi 535 melting point apparatus. Figure 1 shows the IR spectrum of DHME.CH₃SO₃H. The IR spectrum was determined with a Perkin Elmer Spectrum One.

### Example 2

### Preparation of (D)-phenylglycine methyl ester methanesulphonic acid salt (PGM.CH₃SO₃H)

0.5 mol (75.6 g) *(D)*-phenylglycine (ee_{D} >99) was suspended in a solution of 300 ml dimethylcarbonate and 10 ml methanol. Subsequently, 0.7 mol methanesulphonic acid was dosed to the suspension in 30 to 60 min at a temperature increasing from 30 to 75°C. After all the methane sulphonic acid was dosed to the reaction mixture, the temperature was kept at the reflux temperature of dimethylcarbonate, i.e. 84 to 85°C, for 1 hour. The esterification reaction was proceeded for 26 hours at a temperature of 82°C. The conversion of dihydrophenylglycine into dihydrophenylglycine methyl ester after 26 hours at 82°C was 99.3%.

Subsequently, 100 ml of methanol/dimethylcarbonate was distilled off from the reaction mixture at T= 82°C at atmospheric pressure. Thereafter, 200 ml of dimethylcarbonate was added to the reaction mixture and the reaction mixture was slowly cooled down to a temperature of about 20°C. An amount of 0.17 moles of triethylamine was dosed to the reaction mixture in 30 min at 20°C. Subsequently, the reaction mixture was stirred at 20°C for 30 min. The reaction mixture was filtered by G3 filtration and the residue was washed 4 times with 50 ml dimethylcarbonate. The residue consisting of phenylglycine methyl ester methanesulphonic acid salt (PGM.CH₃SO₃H) was dried under vacuum at 60°C under nitrogen atmosphere, for 1 hour.

The yield of *(D)*-phenylglycine methyl ester was 124 g (95%; ee_{D} ≥ 99%).

The melting point of PGM.CH₃SO₃H was 156°C as determined with a Buchi 535 melting point apparatus.

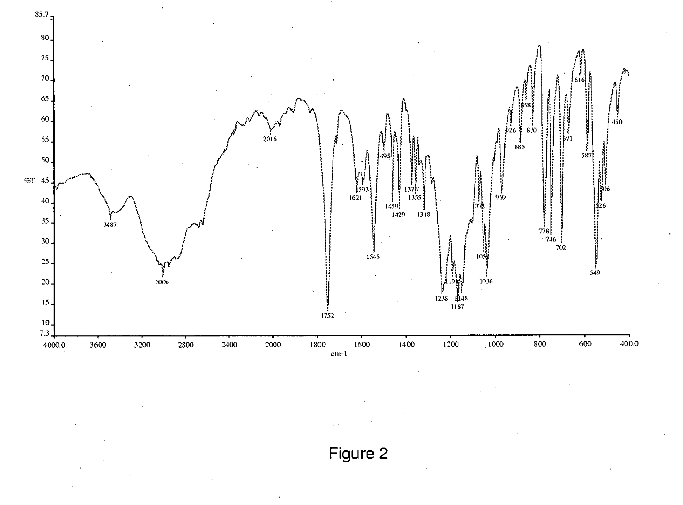

Figure 2 shows the IR spectrum of PGM.CH₃SO₃H. The IR spectrum was determined with a Perkin Elmer Spectrum One.

### Reference Example 3

### Preparation of (D)-phenylglycine methyl ester sulphuric acid salt (PGM.H₂SO₄)

0.5 mole (75.6 g) (D)-phenylglycine (ee_{D} >99) was suspended in a solution of 250 ml dimethylcarbonate. Subsequently, 0.6 mol H₂SO₄ (sulphuric acid) was dosed to the suspension in 30 to 60 min at a temperature increasing from 80 to 90°C. The esterification reaction was proceeded for 20 hours at a temperature of 83°C. The conversion of phenylglycine into phenylglycine methyl ester after 20 hours at 83°C was >99%.

### Reference Example 4

### Preparation of (L)-phenylalanine methyl ester methanesulphonic acid salt

0.25 (41.3 g) mol of *(L)*-phenylalanine was suspended into a solution comprising 150 ml dimethylcarbonate and 5 ml methanol. Subsequently, 0.35 mol of methanesulphonic acid was dosed to the reaction mixture at T = 20°C increasing to T =84°C, within 1 hour. The esterification reaction was proceeded for 24 hour, after which the conversion into *(L)*-phenylalanine methyl ester was 99.4%. The reaction mixture was cooled to T = 25°C and the reaction mixture was subjected to a G3 filtration. The residue was washed 4 times with 25 ml of dimethylcarbonate. The residue was dried under vacuum at 60°C under nitrogen atmosphere, for 1 hour.

### Reference Example 5

### Preparation of 3-amino-3-phenylpropionic acid methyl ester methanesulphonic acid salt

0.25 mol of 3-amino-3-phenylpropionic acid was suspended into a solution comprising 150 ml dimethylcarbonate and 5 ml methanol. Subsequently, 0.35 mol of methanesulphonic acid was dosed to the reaction mixture at a temperature increasing from 30°C to 60°C within 15 min. 20 ml of methanol and 40 ml of dimethylcarbonate was added to the reaction mixture and the reaction mixture was kept for 12 hour at 30°C. The esterification reaction was proceeded for 24 hour at a temperature slowly increasing to 70°C after which the conversion into 3-amino-3-phenylpropionic acid methyl ester was 99.4%. Subsequently, 70 ml of methanol/dimethylcarbonate was distilled off at 80°C and the reaction was proceeded at 84 to 85°C. The reaction mixture was cooled to T = 25°C and the reaction mixture was subjected to a G3 filtration. The residue was washed 4 times with 25 ml of dimethylcarbonate. The residue was dried under vacuum at 60°C under nitrogen atmosphere, for 1 hour.

### Reference Example 6

### Preparation of (D)-α-methyl-phenylglycine methyl ester methanesulphonic acid salt

0.25 ml (41.3 g) *(D)*-α-methyl-phenylglycine was suspended into a solution comprising 150 ml dimethylcarbonate and 5 ml methanol. Subsequently, 0.35 mol of methanesulphonic acid was dosed to the reaction mixture at T = 20°C increasing to T =84°C, within 1 hour. The esterification reaction was proceeded for 48 hour, after which the conversion into D-α-methyl-phenylglycine methyl ester was 97%. The reaction mixture was cooled to T = 25°C and kept at 25°C for 24 hours. Crystallisation was initiated, and subsequently the reaction mixture was subjected to a G3 filtration. The residue was washed 4 times with 25 ml of dimethylcarbonate. The residue was dried under vacuum at 30°C under nitrogen atmosphere, for 1 hour.

### Reference Example 7

### Preparation of ε-amino-capronic acid methyl ester methanesulphonic acid salt

0.25 ml (41.3 g) ε-amino-capronic acid was suspended into a solution comprising 150 ml dimethylcarbonate and 5 ml methanol. Subsequently, 0.35 mol of methanesulphonic acid was dosed to the reaction mixture at T = 20°C increasing to T =84°C, within 20 min. The esterification reaction was proceeded for 20 hours at 84 to 85°C, after which the conversion into ε-amino-capronic acid methyl ester was >99%. The reaction mixture was cooled to T = 20°C. Crystallisation started at 35°C. Subsequently the reaction mixture was subjected to a G3 filtration. The residue was washed 4 times with 25 ml of dimethylcarbonate. The residue was dried under vacuum at 30°C under nitrogen atmosphere, for 1 hour.

### Reference Example 8

### Preparation of methyl benzoate

0.04 mol (5 grams) of benzoic acid was suspended into a solution comprising 50 ml dimethylcarbonate. Subsequently, 0.018 mol of H₂SO₄ was dosed to the reaction mixture at T = 20°C increasing to T =84°C. The esterification reaction was proceeded for 18 hours at 84 to 85°C, after which the conversion into methylbenzoate was >99%.

### Example 9

### Preparation of (D)-phenylglycine methyl ester methanesulphonic acid salt (PGM.CH₃SO₃H) at increased pressure and temperature.

100 g *(D)*-phenylglycine (ee_{D} >99) was suspended in a solution of 390 ml dimethylcarbonate and 13.5 ml methanol. Subsequently, 90 ml methanesulphonic acid was added to the suspension in 30 to 60 min. After the dosage was completed, the reactor was closed and the temperature was increased to 103°C with a concomittant increase of the pressure until 3 kg/cm². The reaction mixture was kept at these conditions for 15 hours while the pressure was maintained at 3 kg/cm² by adjusting the outlet of the CO₂ produced. The conversion of phenylglycine into phenylglycine methyl ester was >99.5% after 15 hours at 103°C and 3 kg/cm². After completion of the reaction time, the overpressure was released and the mixture was cooled down to approximately 20°C. Subsequently, at 20°C, triethylamine was dosed to the reaction mixture to neutralize 85% of the excess of the methanesulphonic acid. The reaction mixture was filtered and the residue was washed with dimethylcarbonate. The yield of *(D)*-phenylglycine methyl ester was - 90% (ee_{D} ≥ 99%).

### FIGURES

Figure 1: IR Spectrum of DHMe.CH₃SO₃H
Figure 2: IR Spectrum of PGM.CH₃SO₃H

## Claims

1. A methanesulphonic acid salt of dihydrophenylglycine methyl ester or phenylglycine methyl ester.

2. Process for the esterification of dihydrophenylglycine or phenylglycine into the corresponding methyl ester comprising bringing said dihydrophenylglycine or phenylglycine into contact with methanesulphonic acid and a solution comprising dialkyl carbonate in a reaction mixture.

3. Process according to claim 2 wherein said solution further comprises methanol.

4. Process according to any one of claims 2 to 3, wherein said esterification comprises keeping the reaction mixture at a temperature below the reflux temperature of the dialkyl carbonate for at least 1 hour.

5. Process according to any one of claims 2 to 4, wherein the pressure is above atmospheric pressure.

6. Process according to any one of claims 2 to 5, wherein said esterification further comprises keeping the reaction mixture at a temperature of at least the reflux temperature of the dialkyl carbonate for at least 6 hours.

7. Process according to any one of claims 2 to 6, wherein said methanesulphonic acid salt of said dihydrophenylglycine or phenylglycine methyl ester is isolated from the reaction mixture.

8. Process according to claim 7 wherein said isolation comprises adding a base to the reaction mixture.

9. Use of a methanesulphonic acid salt of dihydrophenylglycine or phenylglycine methyl ester according to claim 1 in the synthesis of a β-lactam antibiotic.

## Patentansprüche

1. Methansulfonsäuresalz von Dihydrophenylglycinmethylester oder Phenylglycinmethylester.

2. Verfahren zur Veresterung von Dihydrophenylglycin oder Phenylglycin zu dem entsprechenden Methylester, bei dem man das Dihydrophenylglycin oder Phenylglycin mit Methansulfonsäure und einer Dialkylcarbonat umfassenden Lösung in einer Reaktionsmischung in Berührung bringt.

3. Verfahren nach Anspruch 2, bei dem die Lösung ferner Methanol umfasst.

4. Verfahren nach einem der Ansprüche 2 bis 3, bei dem man bei der Veresterung die Reaktionsmischung mindestens 1 Stunde bei einer Temperatur unter der Rückflusstemperatur des Dialkylcarbonats hält.

5. Verfahren nach einem der Ansprüche 2 bis 4, bei dem der Druck über Normaldruck liegt.

6. Verfahren nach einem der Ansprüche 2 bis 5, bei dem man bei der Veresterung ferner die Reaktionsmischung mindestens 6 Stunden bei einer Temperatur, die mindestens der Rückflusstemperatur des Dialkylcarbonats entspricht, hält.

7. Verfahren nach einem der Ansprüche 2 bis 6, bei dem man das Methansulfonsäuresalz des Dihydrophenylglycin- oder Phenylglycinmethylesters aus der Reaktionsmischung isoliert.

8. Verfahren nach Anspruch 7, bei dem man bei der Isolierung eine Base zu der Reaktionsmischung gibt.

9. Verwendung eines Methansulfonsäuresalzes von Dihydrophenylglycin- oder Phenylglycinmethylester nach Anspruch 1 bei der Synthese eines β-Lactam-Antibiotikums.

## Revendications

1. Sel d'acide méthanesulfonique de dihydrophényl-glycine méthylester ou de phénylglycine méthylester.

2. Procédé d'estérification de dihydrophénylglycine ou de phénylglycine en méthylester correspondant, comprenant la mise en contact de ladite dihydrophénylglycine ou phénylglycine avec de l'acide méthanesulfonique et une solution comprenant du carbonate de dialkyle dans un mélange réactionnel.

3. Procédé selon la revendication 2, dans lequel ladite solution comprend en outre du méthanol.

4. Procédé selon l'une quelconque des revendications 2 à 3, dans lequel ladite estérification comprend le maintien du mélange réactionnel à une température inférieure à la température de reflux du carbonate de dialkyle pendant au moins 1 heure.

5. Procédé selon l'une quelconque des revendications 2 à 4, dans lequel la pression est supérieure à la pression atmosphérique.

6. Procédé selon l'une quelconque des revendications 2 à 5, dans lequel ladite estérification comprend en outre le maintien du mélange réactionnel à une température égale à au moins la température de reflux du carbonate de dialkyle pendant au moins 6 heures.

7. Procédé selon l'une quelconque des revendications 2 à 6, dans lequel ledit sel d'acide méthanesulfonique dudit dihydrophénylglycine ou phénylglycine méthylester est isolé à partir du mélange réactionnel.

8. Procédé selon la revendication 7, dans lequel ledit isolement comprend l'addition d'une base au mélange réactionnel.

9. Utilisation d'un sel d'acide méthanesulfonique de dihydrophénylglycine ou phénylglycine méthylester selon la revendication 1, dans la synthèse d'un antibiotique à base de β-lactame.
